(19) Europäisches Patentamt
European Patent Office
Office européen des brevets

(11) Veröffentlichungsnummer: **0 137 440**
**A2**

(12) # EUROPÄISCHE PATENTANMELDUNG

(21) Anmeldenummer: **84111744.3**

(22) Anmeldetag: **02.10.84**

(51) Int. Cl.⁴: **C 07 D 501/46**
A 61 K 31/545

(30) Priorität: **08.10.83 DE 3336756**
**15.03.84 DE 3409431**

(43) Veröffentlichungstag der Anmeldung:
**17.04.85 Patentblatt 85/16**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Fleischmann, Klaus, Dr.**
**Winkelgasse 8**
**D-6500 Mainz 42(DE)**

(72) Erfinder: **Dürckheimer, Walter, Dr.**
**Im Lerchenfeld 45**
**D-6234 Hattersheim am Main(DE)**

(72) Erfinder: **Lattrell, Rudolf, Dr.**
**Heuhohlweg 6H**
**D-6240 Königstein/Taunus(DE)**

(72) Erfinder: **Schwab, Wilfried, Dr.**
**Am Flachsland 18**
**D-6233 Kelkheim (Taunus)(DE)**

(72) Erfinder: **Seeger, Karl, Dr.**
**Schwalbenweg 9**
**D-6238 Hofheim am Taunus(DE)**

(54) **Cephalosporinderivate und Verfahren zu ihrer Herstellung.**

(57) Cephalosporinderivate der allgemeinen Formel

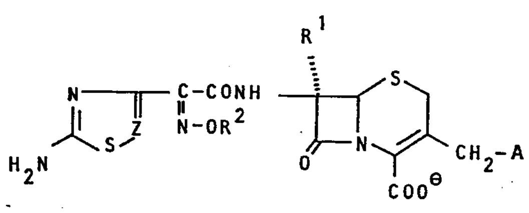

gegen bakterielle Infektionen wirksame pharmazeutische Präparate, die solche Cephemderivate enthalten, Verfahren zur Herstellung der Cephemderivate und der pharmazeutischen Präparate und Verwendung der Cephemderivate zur Bekämpfung bakterieller Infektionen.

EP 0 137 440 A2

Croydon Printing Company Ltd.

0137440

HOECHST AKTIENGESELLSCHAFT HOE 84/F 052 J Dr.KA/mk

# Cephalosporinderivate und Verfahren zu ihrer Herstellung

Die Erfindung betrifft neue Cephalosporinderivate und Verfahren zu ihrer Herstellung, insbesondere polare Cephemderivate, die in 3'-Stellung des Cephemrings durch eine quaternäre Ammoniogruppe substituiert sind und die eine sehr gute antimikrobielle Wirkung gegen gram-positive und gram-negative Bakterien besitzen und deshalb als Arzneimittel zur Behandlung von mikrobiellen Infektionen geeignet sind.

Gegenstand der Erfindung sind daher Cephalosporinderivate der allgemeinen Formel I

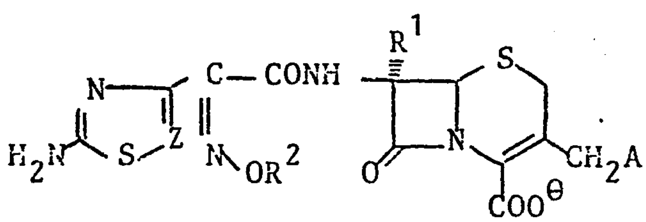

(I)

und deren physiologisch verträgliche Säureadditionssalze, worin

$R^1$ Wasserstoff oder Methoxy,

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-(\overset{R^3}{\underset{R^4}{C}})_m-R^5$, worin m und n jeweils für 0 oder 1 steht

und

$R^3$ und $R^4$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfachsubstituiert sein können,

$R^5$ eine COOH-; CN- oder $CONH_2$-Gruppe,

A eine gegebenenfalls substituierte aliphatische oder cyclische Ammoniogruppe bedeutet,

Z für CH, CF, CCl CBr oder N steht, und in der die $R^2O$-Gruppe in syn-Position steht.

Die vorliegende Erfindung ist insbesondere auf Verbindungen gerichtet, in denen $R^1$ und Z die vorstehende Bedeutung besitzen und

$R^2$=Wasserstoff, $C_1$-$C_6$-Alkyl, das ein- oder mehrfach substituiert sein kann durch Halogen, $C_1$-$C_6$-Alkylthio, $C_1$-$C_6$-Alkoxy, Aryl oder Heteroaryl, $C_2$-$C_6$-Alkenyl, das ein- oder mehrfach substituiert sein kann durch Halogen; $C_2$-$C_3$-Alkinyl, $C_3$-$C_6$-Cycloalkyl, $C_3$-$C_6$-Cycloalkyl-$C_1$-$C_6$-alkyl, $C_4$-$C_7$-Cycloalkenyl und in der die Gruppe

$$(CH_2)_n(\overset{R^3}{\underset{R^4}{C}})_mR^5$$

die vorstehende Bedeutung hat;

A=eine Ammoniogruppe, die von einem tertiären aliphatischen oder 5- bis 7-gliedrigen, vorzugsweise 5- bis 6-gliedrigen cyclischen Amin abgeleitet ist, das durch $C_1$-$C_4$ Alkyl, Hydroxy, $C_1$-$C_4$ Alkyloxy, $C_1$-$C_4$ Alkylthio, Oxo, Halogen, Di-$C_1$-$C_4$-Alkylamino, Trifluormethyl, Cyano, Sulfo, gegebenenfalls substituiertes Carboxy ein- oder mehrfach substituiert sein kann,

gegebenenfalls ein- oder mehrere Doppel- oder Dreifachbindungen enthalten sein können und bei cyclischen Aminen ein oder mehrere C-Atom der Ringe durch Heteroatome, wie z.B.: O,S,N, ersetzt sein können, und wobei auch bei diesen bevorzugten, unter die allgemeine Formel I fallenden Verbindungen die $R^2O$-Gruppe in syn-Position steht.

Als besonders bevorzugt kommen beispielsweise die folgenden Substituenten in Betracht:

$R^1$ = Wasserstoff oder Methoxy

$R^2$ = Wasserstoff, $C_1$-$C_6$-Alkyl, wie z.B.: Methyl, Ethyl, Propyl, Isopropyl, Butyl, vorzugsweise Methyl und Ethyl; $C_1$-$C_4$-Halogenalkyl, z.B. Fluor-, Chlor-, Brom- oder Jod-substituiertes Alkyl, vorzugsweise Difluormethyl, Trifluorethyl, 2,2,3,3-Tetrafluorpropyl; $C_1$-$C_2$-Alkyloxy-$C_1$-$C_2$-alkyl, z.B.: Methoxymethyl, Äthoxyäthyl; $C_1$-$C_2$-Alkylmercapto-$C_1$-$C_2$-alkyl, z.B.: Methylmercaptomethyl; durch Aryl, wie z.B. Phenyl, Tolyl, Chlorphenyl, substituiertes Alkyl, insbesonders Benzyl;
durch Heteroaryl substituiertes Alkyl, wie z.B. 1,3-Thiazol-4-yl-substituiertes Alkyl, insbesondere 1,3-Thiazol-4-yl-methyl;
$C_2$-$C_6$-Alkenyl, wie z.B. Vinyl, Allyl, Isopropenyl, Methallyl, isnbesondere Allyl, Methallyl;
durch Halogen, wie z.B. durch Chlor oder Brom substituiertes $C_2$-$C_6$-Alkenyl, insbesondere 3-Chlorpropen-2-yl, 2-Brom-propen-2-yl, 2-Chlorpropen-2-yl;
$C_2$-$C_3$-Alkinyl, wie insbesondere Propargyl;
$C_3$-$C_7$-Cycloalkyl, wie insbesondere Cyclopropyl, Cyclobutyl, Cyclopentyl;
$C_3$-$C_7$-Cycloalkyl-$C_1$-$C_6$-alkyl, wie insbesondere Cyclopropylmethyl, Cyclobutylmethyl;
$C_4$-$C_7$-Cycloalkenyl, wie insbesondere Cyclopenten-1-yl;

die Gruppe $-(CH_2)_n-(\overset{R^3}{\underset{R^4}{C}})_m-R^5$, wobei $R^5$ die Gruppe COOH, CN oder $CONH_2$ bedeutet und $R^3$ und $R^4$ gleich oder verschieden sein können und Wasserstoff, Aryl, vorzugsweise Phenyl; $C_1$-$C_4$-Alkyl, wie z.B. Methyl, Ethyl, Propyl, Isopropyl, Butyl, sec. Butyl, vorzugsweise Methyl, Ethyl, insbesondere Methyl, bedeuten können oder

wobei $R^3$ und $R^4$ zusammen mit dem Kohlenstoffatom, an das sie gebunden sind, eine Methylengruppe oder eine $C_3$-$C_7$-Cycloalkylidengruppe bilden können, wie z.B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl, und wobei die Cycloalkylidengruppe substituiert sein kann, z.B. durch $C_1$-$C_4$-Alkyl, vorzugsweise Methyl, oder durch Halogen vorzugsweise Fluor und Chlor,

m = 0 oder 1

n = 0 oder 1, wobei die Summe von m und n 1 oder 2 darstellt.

Bevorzugte Beispiele für die Gruppe- $(CH_2)_n(\overset{R^3}{\underset{R^4}{C}})_m$- sind die folgenden:

Für den Fall, daß n = 0 und m = 1 ist;
$CH(CH_3)$, $C(CH_3)_2$, $CH(C_6H_5)$

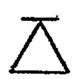 , 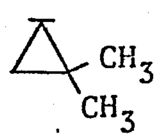 , 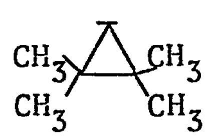 , 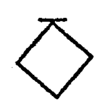 ,

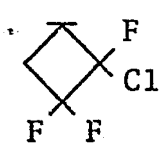 , 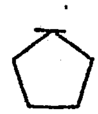 , 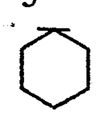 ,

für den Fall, daß m = 0 und n = 1 ist: $-CH_2-$ und falls n und m = 1 sind: $-CH_2-C(CH_2)-$.

A= Ammonio, das von einem tertiären organischen aliphatischen oder cyclischen Amin abgeleitet ist, beispielsweise $C_1$-$C_6$-Trialkylammonio, insbesondere Trimethylammonio-, Triäthylammonio-, Tripropylammonio-, Dimethyläthylammonio-, Diäthylmethylammonio-, Dimethylbenzylammonio-, Dimethylpropagylammonio-, wobei die Alkylreste auch zu einem oder zwei Ringen mit jeweils 2-7 C-Atomen verknüpft sein können, wie z.B.: 1-Methylpyrrolidinio-, 1-Methylpiperidino-, Chinuclidinio- und wobei in den Ringen auch ein oder zwei Doppelbindungen enthalten sein können, wie z.B.: 1-Methyl-1,1,5,6-Tetrahydropyridinio und weiterhin auch ein oder mehrere Ring-C-Atome durch Heteroatome, vorzugsweise N, O oder S ersetzt sein können, wie beispielsweise N-Methylmorpholinio-, 1,4-Dimethylpiperazino-, 1-Ammonio-4-azabicyclo(2,2,2)octan oder 1-Ammonio-4,6-dioxabicyclo(3,3,0) octan.

Die A zugrunde liegenden tertiären Amine können weiterhin ein- oder mehrfach, gleich oder verschieden substituiert sein durch $C_1$-$C_4$-Alkyl, wie insbesondere Methyl, Äthyl, beispielsweise 1,2-Dimethylpyrrolidinio-;

Hydroxy, wie beispielsweise N,N'-Diäthyläthanolammonio-, N-Äthyldiäthanolammonio-, Triäthanolammonio-, 4-Hydroxy-1-methylpiperidinio-, N-(ß-Hydroxyäthyl)-morpholinio-;

$C_1$-$C_4$-Alkyloxy, insbesondere Methyloxy und Äthyloxy, wie beispielsweise N-Methoxyäthyl-dimethylammonio-, N-Methoxyäthyl-morpholinio-;

$C_1$-$C_4$-Alkylthio, insbesondere Methylthio und Äthylthio;

Oxo, wei beispielsweise 1-Methyl-4-oxo-piperidinio;

Halogen, insbesondere Chlor, wie z.B.: 4-Chlor-1-methylpiperidinio;

$C_1$-$C_4$-Alkyloxycarbonyl, insbesondere Methyloxycarbonyl, Äthyloxycarbonyl, wie beispielsweise 1-Methyl-4-äthoxycarbonylpiperidinio;

Dialkylammino, Trifluormethyl, Cyano, Sulfo, Sulfoniederalkyl, Carboxy und Carbamoyl.

Das erfindungsgemäße Verfahren zur Herstellung von Verbindungen der Formel I ist dadurch gekennzeichnet, daß man

a) eine Verbindung der allgemeinen Formel II

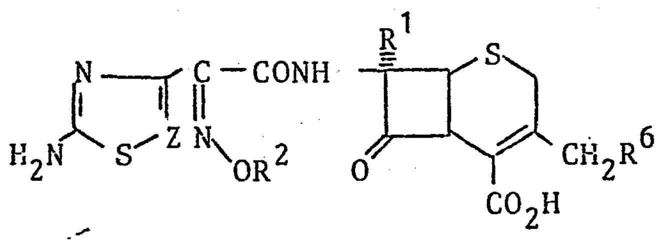

(II)

oder deren Salze, worin $R^1$, $R^2$ und Z die in Formel I genannte Bedeutung haben und $R^6$ eine durch diejenige Base, die den Resten A der Formel I entsprechen, austauschbare Gruppe bedeutet, mit dieser Base umsetzt und

α) eine gegebenenfalls vorhandene Schutzgruppe abspaltet und

ß) falls erforderlich, das erhaltene Produkt in ein physiologisch verträgliches Säureadditionssalz überführt.

oder

b) eine Verbindung der allgemeinen Formel III

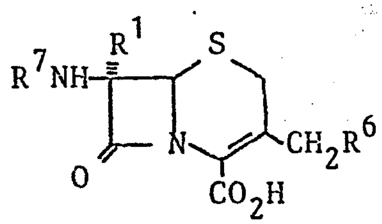

(III)

worin $R^1$ und $R^6$ die vorstehend für die Formel II genannte Bedeutung haben und $R^7$ für Wasserstoff oder eine Aminoschutzgruppe steht, mit der Base, die dem in Formel I definierten Rest A zugrunde liegt, umsetzt unter Bildung der Verbindung der allgemeinen Formel IV,

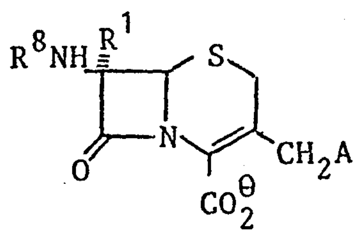

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

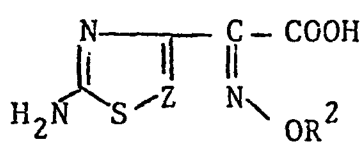

(V)

worin $R^2$ und Z die genannte Bedeutung besitzen, oder mit einem an der Carbonylgruppe aktivierten Derivat dieser Verbindung umsetzt und

α) eine gegebenenfalls vorhandene Schutzgruppe abspaltet und

ß) falls erforderlich, das erhaltene Produkt der allgemeinen Formel I in ein physiologisch verträgliches Säureadditionssalz überführt.

Soll die Herstellung der Verbindungen der allgemeinen Formel I durch nucleophilen Austausch von $R^6$ in den Verbindungen der allgemeinen Formel II mit der Base, die dem in Formel I definierten Rest A zugrunde liegt, erfolgen, so kommen als Reste $R^6$ insbesondere in Betracht Acyloxyreste von niederen aliphatischen Carbonsäuren, vorzugsweise mit 1-4 C-Atomen, wie z.B.: Acetoxy, die gegebenenfalls substituiert sein können, wie z.B.: Chloracetoxy oder Dichloracetoxy, oder Carbamoyloxy oder ein Halogenatom wie z.B. Chlor, Brom oder Jod.

Die nucleophile Austauschreaktion an Verbindungen der allgemeinen Formel II, kann so erfolgen, daß die Reaktion in Gegenwart der den Resten A entsprechenden Base und von Tri-$C_1$-$C_4$-alkyljodsilanen, wie z.B. Trimethyl- oder Triäthyljodsilan, vorgenommen wird. Dabei kann so verfahren werden, daß die Verbindung II wobei $R^6$ für Acetoxy steht, zuerst mit Trimethyljodsilan nach den im folgenden genannten Reaktionsbedingungen zur Reaktion gebracht wird, die gebildete Verbindung II mit $R^6$ = J isoliert und anschließend mit der Base umgesetzt wird, oder die 3-$CH_2$J-Verbindung in situ durch Zugabe der Base zur Reaktion gebracht wird. Eine bevorzugte Ausführungsform ist in den deutschen Patentanmeldungen P 3316796.6, P 3316797.4 und P 3316798.2 beschrieben. Sie besteht darin, daß zu einer Lösung oder Suspension der Verbindung II in einem geeigneten Lösungsmittel die dem Rest A entsprechende Base zugegeben wird, gefolgt von Trimethyljodsilan. Statt Trimethyljodsilan kann beispielsweise auch eine Reaktionsmischung aus Jod und Hexamethyldisilan, die vorher bei Temperaturen zwischen etwa 60 und 120 °C

in literaturbekannter Weise zur Reaktion gebracht wurden, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.

Die Reaktion wird ausgeführt bei Temperaturen zwischen etwa -5° und +100 °C, vorzugsweise zwischen +10 °C und +80°C.

Geeignete inerte aprotonische Lösungsmittel sind z.B. chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril oder Frigene; insbesondere ist Methylenchlorid ein hervorragendes Lösungsmittel.

Die dem Rest A entsprechende Base wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 2-5 Mol der Base für die Substitution zur Verfügung stehen.

Da neben der auszutauschenden Gruppe $R^6$ in der Ausgangsverbindung II auch andere funktionelle Gruppen, wie z.B. die Carboxylgruppe, mit Trimethyljodsilan reagieren, wird letzteres in mindestens doppeltem bis zu fünfzehnfachen, vorzugsweise in drei- bis zehnfachem Überschuß zugegeben.

Derartige funktionelle Gruppen können auch durch Zugabe eines Silylierungsmittels wie z.B. Bistrimethylsilylacetamid, N-Methyl-N-trimethylsilyltrifluoracetamid; Bistrimethylsilyltrifluoracetamid,

Trimethylchlorsilan, Hexamethyldisilazan, Bistrimethylsilylharnstoff, vorsilyliert werden, entweder ohne oder in Gegenwart einer Base, vorzugsweise der gewünschten, der Gruppe A zugrundeliegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Die Reaktionsprodukte der Formel I können beispielsweise nach Zugabe von Wasser oder wäßrigen Mineralsäuren, z.B. verdünnter HCl, HBr, HJ oder $H_2SO_4$, aus der wäßrigen Phase in üblicher Weise, z.B. durch Gefriertrocknen der Wasserphase, Chromatographie, oder Fällung durch Zugabe von organischen Lösungsmitteln, isoliert werden. Vorzugsweise werden die Reaktionsprodukte durch Ausfällen aus der wäßrigen Lösung in Form eines schwerlöslichen Salzes, beispielsweise eines Hydrojodidsalzes, isoliert.

Für den Fall, daß $R^6$ für eine Carbamoyloxygruppe steht, wird die Austauschreaktion analog durchgeführt. Steht $R^6$ für Brom, so erfolgt der Austausch in literaturbekannter Weise.

Nach der Verfahrensvariante b) werden die Verbindungen der allgemeinen Formel I durch Acylierung von Verbindungen der allgemeinen Formel IV oder deren Additionssalze, beispielsweise mit Chlorwasserstoffsäure, Bromwasserstoffsäure, Salpetersäure, Schwefelsäure, Phosphorsäure oder einer organischen Säure, wie z.B. Methansulfonsäure oder Toluolsulfonsäure, mit Carbonsäuren der allgemeinen Formel V oder mit einem reaktionsfähigen Derivat einer solchen Säure durchgeführt.

Es ist dabei nicht erforderlich, die Verbindungen der allgemeinen Formel IV zu isolieren. Die Reaktion

kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren reaktionsfähige Derivate in einem geeigneten Lösungsmittel mit der dem Rest A in der allgemeinen Formel IV entsprechenden Base umsetzt und die entstandene Verbindung der allgemeinen Formel IV in situ zu den Verbindungen der allgemeinen Formel I acyliert. Erfindungsgemäß werden bei dieser Reaktion Ausgangsverbindungen der allgemeinen Formel III, in denen $R^6$ für Jod steht, eingesetzt. Geeignete Lösungsmittel sind chlorierte Kohlenwasserstoffe, wie z.B.: Methylenchlorid und Chloroform; Ether, wie z.B.: Diethylether, Tetrahydrofuran, Dioxan; Acetonitril und Amide, wie vorzugsweise Dimethylformamid und Dimethylacetamid. Es kann sich auch als vorteilhaft erweisen, Gemische der genannten Lösungsmittel zu verwenden.

Liegt in den Verbindungen der allgemeinen Formel III ein reaktionsfähiges Derivat vor, so kommen insbesondere Silylderivate in Betracht, die bei der Umsetzung von Verbindungen der allgemeinen Formel III mit Silylverbindungen, wie z.B.: Trimethylchlorsilan oder Bis-(trimethylsilyl)-acetamid gebildet werden. Die dem Rest A entsprechende Base wird in mindestens stöchiometrischer Menge, bis zu einem zehnfachen Überschuß, vorzugsweise 2 bis 5 Äquivalente verwendet. Die Reaktion wird bei Temperaturen zwischen etwa -5 und +100 °C, vorzugsweise zwischen +20 und +50 °C ausgeführt.

Die Verbindungen der allgemeinen Formel IV können auch aus Verbindungen der allgemeinen Formel III, wobei $R^6$ für Acetoxy steht, in analoger Weise wie vorstehend für die Verbindungen der allgemeinen Formel II beschrieben, dargestellt werden.

Werden die Carbonsäuren der allgemeinen Formel V sowie ihre an der Aminogruppe geschützten Derivate selbst als Acylierungsmittel eingesetzt, so wird zweckmäßig in Gegenwart eines Kondensationsmittels, beispielsweise eines Carbodiimds, wie beispielsweise N,N'-Dicyclohexylcarbodiimid, gearbeitet.

Die Aktivierung von Carbonsäuren der allgemeinen Formel V kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie z.B. in der deutschen Patentschrift 28 04 040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmittel, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel V eignen sich ferner die Anhydride und gemischten Anhydride, Azide, aktivierten Ester und Thioester. Als gemischte Anhydride sind besonders geeignet solche mit niederen Alkansäuren, wie z.B. Essigsäuren und besonders bevorzugt solche mit substituierten Essigsäuren, wie z.B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel V, in denen die Aminogruppe geschützt ist, mit Chlorameisensäurebenzylester, -p-nitrobenzylester, -iso-butylester, -ethylester oder allylester gewinnt.

Als aktivierte Ester eignen sich vorzugsweise diejenigen mit p-Nitrophenol, 2,4-Dinitrophenol, Methylcyanhydrin, N-Hydroxysuccinimid und N-Hydroxyphtalimid, insbesondere diejenigen mit 1-Hydroxybenzotriazol und 6-Chlor-1-hydroxybenzotriazol. Besonders bevorzugte Thioester sind beispielsweise diejenigen mit 2-Mercaptobenzothiazol und 2-Mercaptopyridin. Die aktivierten Derivate können als isolierte Substanzen aber auch in situ umgesetzt werden.

Im allgemeinen erfolgt die Umsetzung der Cephemderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther, vorzugsweise Tetrahydrofuran und Dioxan; Ketone, wie vorzugsweise Aceton und Butanon; Amide, wie vorzugsweise Dimethylformamid und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Die Umsetzung von Cephemverbindungen der Formel IV mit Carbonsäuren der Formel V bzw. deren aktivierten Derivaten kann in einem Temperaturbereich von etwa -80 bis etwa +80 °C, vorzugsweise zwischen etwa -20 °C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen

1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z.B. Triäthylamin oder Dimethylanilin, anorganische Basen, wie z.B. Kaliumcarbonat oder Natriumcarbonat, Alkylenoxide, wie z.B. Propylenoxid. Auch die Anwesenheit eines Katalysators, wie z.B. von Dimethylaminopyridin, kann gegebenenfalls von Vorteil sein. Liegt in den Verbindungen der allgemeinen Formel IV die Aminogruppe in Form eines reaktionsfähigen Derivats vor, so kann es sich um ein solches handeln, wie aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z.B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid durchzuführen.

Als physiologisch verträgliche Säureadditionssalze der Verbindung der allgemeinen Formel I seien beispielsweise erwähnt solche mit Chlorwasserstoffsäure, Bromwasserstoffsäure, Salpetersäure, Phosphorsäure, Schwefelsäure oder organische Säuren, wie z.B. Methansulfonsäure, p-Toluolsulfonsäure oder Maleinsäure.

Die Verbindungen der allgemeinen Formel II und III sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombinationen mit anderen Wirkstoffen, beispielsweise aus der Reihe der Penicilline, Cephalosporine oder Aminoglykoside zur Anwendung kommen.

Die Verbindungen der allgemeinen Formel I und ihre physiologischen verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel mit mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Puffersubstanzen sind beispielsweise organische Verbindungen, wie z.B. N,N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen, wie z.B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Einige Verbindungen der allgemeinen Formel I, in der $R^1$ für Wasserstoff und Z für CH steht, sind aus der Literatur bekannt und zwar

α) für

$$R^2 = \begin{array}{c} R^a \\ | \\ -C-COOH \\ | \\ R^b \end{array}$$

mit $R^a$ und $R^b$ = $C_1$-$C_4$-Alkyl oder $C_3$-$C_7$-Cycloalkyliden und A = Tri-$C_1$-$C_4$-Alkylammonium aus der DE-OS 29 43 437 oder

β) für $R^2$ = $C_1$-$C_4$-Alkyl, Allyl, 2-Butenyl, 3-Butenyl oder die Gruppe

$$R'-\underset{\mathrm{COOH}}{\underset{|}{\overset{|}{C}}}-R''$$

worin R' und R" für Wasserstoff, Methyl oder Äthyl oder auch zusammen für $C_3$-$C_5$-Cycloalkyliden steht und A 1-Methyl-1-pyrrolidinium bedeutet, aus der DE-OS 33 07 550.

Die folgenden Ausführungsbeispiele für erfindungsgemäß herstellbare syn-Verbindungen dienen zur weiteren Erläuterung der Erfindung, schränken sie jedoch nicht darauf ein.

Beispiel 1

7-[2-(2-Aminothiazol-4-yl)-2-syn-methoxyimino-acetamido]-3-triäthylammoniomethyl-ceph-3-em-4-carboxylat

Verfahren a)

2.28 g (5 mmol) 7-[2-(2-Aminothiazol-4-yl)-2-syn-methoxyimino-acetamido]-cephalosporansäure werden in 10 ml Chloroform suspendiert und nach Zugabe von 3 g (2.8 ml, 15 mmol) N-Methyl-N-trimethylsilyltrifluoracetamid 1.5 Stunden bei Raumtemperatur bis zur vollständigen Lösung gerührt. Sodann werden 2.8 g (1.9 ml, 14 mmol) Trimethyljodsilan zugegeben und weitere 15 Minuten gerührt. Das Lösungsmittel wird i. Vak. entfernt, der harzige Rückstand in 10 ml Acetonitril gelöst. Bei 0-5° C werden nacheinander 0.2 ml Wasser und 1.4 ml (10 mmol) Triäthylamin, gelöst in 3 ml Acetonitril, zugegeben und die Mischung sodann ohne Kühlung 2 Stunden bei Raumtemperatur gerührt. Sodann werden 3 g Eis zugegeben und die dunkelgefärbte Lösung i. Vak. auf ein Volumen von ca. 5 ml eingeengt. Durch Zugabe von Natriumbicarbonat wird auf pH 6 gestellt, von wenig Ungelöstem filtriert und das Filtrat über Kieselgel ("Lobar-C"-Fertigsäule, Fa. Merck, Art. 10402) mit Aceton/Wasser (3:1) chromatographiert. Nach Gefriertrocknung der Produktfraktionen erhält man 0.98 g (39.5 % d. Th.) der Titelverbindung als farblosen amorphen Feststoff.

$^{1}$H-NMR ($CF_3CO_2D$): δ = 1.2-1.8 (m, 9H, $CH_2\underline{CH_3}$), 3.1-3.7 (m, 6H, $\underline{CH_2}CH_3$), 3.5-3.8 (m, 2H, $SCH_2$), 4.25 (s, 3H, $OCH_3$), 4.52 und 4.86 ($AB_q$, J=14Hz, 2H, $CH_2N^{\oplus}$), 5.48 und 5.91 ( je ein d, J=5Hz, 2 Lactam-H), 7.42 ppm (s, 1H, Thiazol-H).

Analog Beispiel 1 werden die nachfolgend aufgeführten Verbindungen als amorphe Feststoffe erhalten, die der allgemeinen Formel I mit $R^1$ = Wasserstoff und $R^2$ = Methyl entsprechen und worin Z und A die in Tabelle 1 angegebene Bedeutung haben.

Tabelle 1

| Beispiel | Z | A | $^1$H-NMR in $CF_3CO_2D$: δ (ppm) = |
|---|---|---|---|
| 2 | CH | 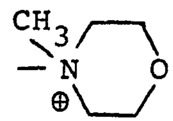 | 3.48 (s, 3H, $CH_3$), 3.55-4.38 (m, 10H, $SCH_2$ und 8 Morpholin-H), 4.26 (s, 3H, $OCH_3$), 4.82 und 5.05 (ABq, J=14Hz, 2H, $CH_2N^{\oplus}$), 5.50 und 5.93 (je ein d, J=5Hz, 2 Lactam-H), 7.42 (s, 1 Thiazol-H). |
| 3 | CH | 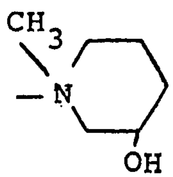 | 1.7-2.6 (m, 4H, Piperidin), 2.9-4.7 (m, 8H mit s bei 3.36, $CH_3$ und Piperidin-H), 4.25 (s, 3H, $OCH_3$), 4.78-5.95 (m, 4H, $CH_2N^{\oplus}$ und 2 Lactam-H), 7.42 (s, 1 Thiazol-H). |
| 4 | CH | 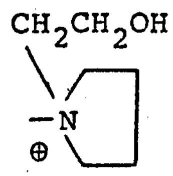 | 2.05-2.65 (m, 4 Pyrrolidin-H), 2.8-4.5 (m, 10H, $SCH_2$, $CH_2CH_2OH$, 4 Pyrrolidin-H), 4.25 (s, 3H, $OCH_3$), 4.8-5.1 (m, 2H, $CH_2N^{\oplus}$), 5.44 und 5.92 (je ein d, J=5Hz, 2 Lactam-H, 7.42 (s, 1 Thiazol-H). |
| 5 | N | $-\overset{\oplus}{N}(CH_3)_3$ | 3.46 (s, 9H, $CH_3$), 3.77 und 3.89 ($AB_q$, J=19Hz, 2H, $SCH_2$), 4.43 (s, 3H, $OCH_3$), 4.81 und 4.93 ($AB_q$, J=14Hz, 2H, $CH_2N^{\oplus}$), 5.48 und 5.95 (je ein d, J=5Hz, 2 Lactam-H). |

## Beispiel 6

### 7-[2-(2-Aminothiazol-4-yl)-syn-methoxyimino-acetamido]-3-(tri-n-propylammonio-methyl)-ceph-3-em-4-carboxylat

Verfahren b

Lösung A

1.02 g (3 mmol) 7-Amino-3-jodmethyl-ceph-3-em-4-carbonsäure werden in 50 ml N,N-Dimethylformamid suspendiert, 1.29 g (9 mmol) Tri-n-propylamin zugesetzt und 2 Stunden bei Raumtemperatur gerührt (DC-Kontrolle).

Lösung B

0.8 g (4 mmol) 2-(2-Aminothiazol-4-yl)-2-syn-methoxyimino-essigsäure werden zusammen mit 0.61 g (4 mmol) 1-Hydroxy-1H-benzotriazol-Hydrat und 0.865 g (4.2 mmol) N,N'-Dicylohexylcarbodiimid in 15 ml Dimethylformamid gelöst, 3 Stunden gerührt und vom ausgefallenen Dicyclohexylharnstoff abfiltriert.

Die Lösung B wird bei 0° C zur Lösung A getropft, dann 4 Stunden bei Raumtemperatur gerüht (DC-Kontrolle) und i. Vak. eingeengt. Man nimmt mit wenig Aceton/Wasser (3:1) auf und chromatographiert mit demgleichen Laufmittel an Kieselgel ("Lobar-C"-Säule). Gefriertrocknung der Produktfraktionen liefert einen nahezu farblosen, amorphen Feststoff. Ausbeute: 0.44 g.

$^1$H-NMR ($CF_3CO_2D$): δ = 1.80-1.40 (t, 9H, J=6Hz, $NProp_3$), 1.50-2.35 (m, 6H, $NProp_3$), 3.0-3.65 (m, 6H, $NProp_3$), 3.76 (br. s, 2H, $SCH_2$), 4.23 (s, 3H, $OCH_3$), 4.40-5.10 ($AB_q$, 2H, J=14Hz, $CH_2N^{\oplus}$), 5.46 und 5.96 (jew. d, J=5Hz, Lactam-H), 7.43 (s, 1H, Thiazol).

Analog Beispiel 6 werden die nachfolgend aufgeführten Beispiele als amorphe Feststoffe erhalten, die der allgemeinen Formel I mit $R^1$= Wasserstoff und Z= CH entsprechen und worin $R^2$ und A die in Tabelle 2 angegebene Bedeutung haben.

Tabelle 2

| Beispiel | $R^2$ | A | $^1$H-NMR in $CF_3CO_2D$ : δ (ppm) = |
|---|---|---|---|
| 7 | Me | Me<br>$-N^{\oplus}-Et$<br>Me | 1.58 (bt, J=6Hz, 3H, NEt), 3.26 (bs, 6H, $NMe_2$), 3.5-3.86 (4H, q von NEt bei 3.66 überlagert mit bs von $SCH_2$ bei 3.8), 4.28 (s, 3H, OMe), 4.42-5.13 (AB, J=14Hz, 2H, $CH_2N^{\oplus}$), 5.47 und 5.93 (je ein d, J=4.5Hz, 2 Lactam-H), 7.44 (s, 1H, Thiazol). |
| 8 | Me | Me<br>$-N^{\oplus}-CH_2CH_2OH$<br>Me | 3.2-4.55 (m, ~ 15H, $-N-CH_2CH_2$ überlagert mit $NMe_2$ (br. s bei 3.4), $SCH_2$(3.86) und OMe ( s bei 4.28), 4.6-5.3 (AB, J=15Hz, 2H, $CH_2N^{\oplus}$), 5.49 und 5.94 (je ein d, J=5Hz, 2 Lactam-H), 7.46 (s, 1H, Thiazol). |
| 9 | Me | Me<br>$-N^{\oplus}-CH_2-C≡CH$<br>Me | 3.1 (b. s, 1H, C≡CH), 3.4 (bs. s, 6H, $NMe_2$), 3.85 (s, 2H, $SCH_2$), 4.26 ( s, 3H, OMe), 4.36 (br. s, 2H, $N-CH_2C≡C$), 4.51-5.23 (AB, J=14Hz, 2H, $CH_2N^{\oplus}$), 5.5 und 5.93 ( jew. d, J=4.5Hz, 2 Lactam-H), 7.43 (s, 1H, Thiazol). |

Tabelle 2 (Forts.)

| Beispiel | $R^2$ | A | $^1H$-NMR in $CF_3CO_2D$: δ (ppm) = |
|---|---|---|---|
| 10 | Me | 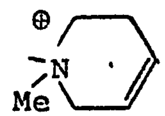 | 2.5-5.1 (m,~ 16H, dreimal $CH_2$ von Dehydropiperidin überlagert mit N-Me (br. s bei 3.28), $CH_2S$ (br. s bei 3.83) und OMe (s bei 4.25), sowie AB von $CH_2N^{\oplus}$ (~4.5-5.1), 5.35-6.35 (m, 4H, 2 Lactam-H und 2 Olefin-H), 7.39 (s, 1H, Thiazol). |
| 11 | Me | 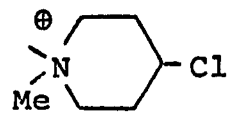 | 2.03-2.8 (m, 4H, zweimal $CH_2$ von Piperidin), 3.1-5-15 (m,~ 15H, Piperidin-H, überlagert mit NMe (bs bei 3.33), $SCH_2$ (bs bei 3.86), OMe (s bei 4.27), sowie AB von $CH_2N^{\oplus}$ (4.5-5.15, J=14Hz), 5.5 und 5.93 (jew. d, J=4.5Hz, 2 Lactam-H), 7.43 (s, 1H, Thiazol). |
| 12 | Me | 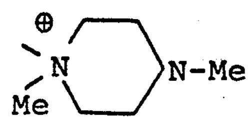 | 3.3 und 3.63 (jew. bs, 6H, zweimal NMe), 3.7-4.4 (m,~ 10H, Piperazin-H, überlagert mit $SCH_2$ (3.85) und OMe (s bei 4.25), 5.03 (bs, 2H, $CH_2N^{\oplus}$), 5.46 und 5.9 (jew. d, J=4.5Hz, 2 Lactam-H), 7.41 (s, 1H, Thiazol). |

Tabelle 2 (Forts.)

| Beispiel | $R^2$ | A | $^1H$-NMR in $CF_3CO_2D$: δ (ppm) = |
|---|---|---|---|
| 13 | Me | $-\overset{\oplus}{N}Et_3$ | NMR identisch mit Beispiel 1 |
| 14 | Me | $-\overset{\oplus}{N}Me_3$ | 3.4 (s, 9H, $NMe_3$), 3,83 (bs, 2H, $SCH_2$), 4.30 (s, 3H, OMe), 4.50-5.10 (ABq, J=14Hz, 2H, $CH_2N^{\oplus}$), 5.50 und 5.95 (je ein d, J=5Hz, 2 Lactam-H), 7.45 (s, 1H, Thiazol). |
| 15 | Et | $-\overset{\oplus}{N}Me_3$ | 1.46 (t, J=7Hz, 3H, OEt), 3.36 (s, 9H, $NMe_3$), 3.83 (bs, 2H, $SCH_2$), 4.53 (q, J=7Hz, 2H, OEt), 4.50-5.10 (ABq, J=14Hz, 2H, $CH_2N^{\oplus}$), 5.50 und 5.96 (jew.d, J=5Hz, 2 Lactam-H), 7.43 (s, 1H, Thiazol). |
| 16 | Et | $-\overset{\oplus}{N}Et_3$ | 1.23-1.8 (m, 12H, OEt und $NEt_3$), 3.2-4.0 (m, 8H, $SCH_2$ und $NEt_3$), 4.56 (q, J=7Hz, OEt), 4.50-5.05 (ABq, J=14Hz, 2H, $CH_2N^{\oplus}$), 5.30 und 5.96 (jew. d, J=5Hz, 2 Lactam-H), 7.43 (s, 1H, Thiazol). |
| 17 | $CH_2CF_3$ | $-\overset{\oplus}{N}Me_3$ | 3.40 (s, 9H, $NMe_3$), 3.80 (bs, 2H, $SCH_2$), 4.30-5.0 (m, 4H, $CH_2N^{\oplus}$ und $CH_2CF_3$), 5.50 und 5.90 (jew. d, J=5Hz, 2 Lactam-H), 7.43 (s, 1H, Thiazol). |

Tabelle 2 (Forts.)

| Beispiel | $R^2$ | A | $^1H$-NMR in $CF_3CO_2D$: δ (ppm) = |
|---|---|---|---|
| 18 | $CH_2CF_3$ | $-\overset{\oplus}{N}Et_3$ | 1.53 (bt, J=6Hz, 9H, $NEt_3$), 3.20-3.90 (m, 8H, $NEt_3$ und $SCH_2$), 4.40-5.10 (m, 4H, $CH_2N^{\oplus}$ und $CH_2CF_3$), 5.50 und 5.73 (jew. d, J=5Hz, 2 Lactam-H), 7.43 (s, 1H, Thiazol). |
| 19 | $CHF_2$ | $-\overset{\oplus}{N}Et_3$ | 1.53 (bt, J=6Hz, 9H, $NEt_3$), 3.15-3.95 (m, 8H, $SCH_2$ und $NEt_3$), 4.5-4.9 (m, 2H, $CH_2N^{\oplus}$), 5.46 und 5.9 (jew. d, J=5Hz, 2 Lactam-H), 6.73 (t, J=72Hz, 1H, $CHF_2$), 7.5 (s, 1H, Thiazol). |
| 20 | Me | 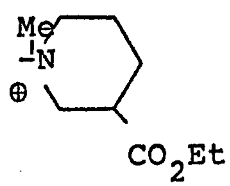 | 1.40 (t, J=7Hz, 3H, OEt), 1.70-2.70 (m, 4H, Piperidin-H), 3.0-4.10 (m, 9H, $SCH_2$, NMe und Piperidin-H), 4.26 (s, 3H, OMe), 4.30-5.10 (m, 3H, $CH_2N^{\oplus}$ und C<u>H</u>-COOEt), 5.46 und 5.96 (jew. d, J=5Hz, 2 Lactam-H), 7.43 (s, 1H, Thiazol). |
| 21 | Et | 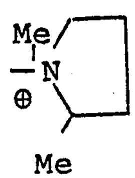 | 1.33-1.66 (m, 6H, OEt und CH-$CH_3$), 2.0-2.90 (m, 4H, Pyrrolidin-H), 3.0-3.45 (m, 6H, NMe und 3 Pyrrolidin-H), 3.80 (bs, 2H, $SCH_2$), 4.20-4.95 (m, 4H, $CH_2N^{\oplus}$ und OEt), 5.50 und 5.93 (jew. d, J=5Hz, 2 Lactam-H), 7.43 (s, 1H, Thiazol) |

Tabelle 2 (Forts.)

| Beispiel | $R^2$ | A | $^1$H-NMR ($CF_3CO_2D$): δ (ppm) = |
|---|---|---|---|
| 22 | Et | 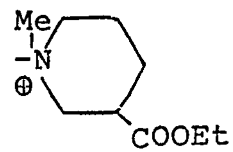 | 1.26-1.60 (m, 6H, COOEt und OEt), 1.90-2.50 (m, 4H, Piperidin-H), 3.0-5.0 (m, 13H, $SCH_2$, $CH_2N^{\oplus}$, NMe, OEt und 4 Piperidin-H), 5.46 und 6.0 (jew. d, J=5Hz, 2 Lactam-H), 7.43 (s, 1H, Thiazol). |
| ·23 | $CHF_2$ | $\overset{\oplus}{N}Me_3$ | 3.40 (s, 9H, $NMe_3$), 3.83 (bs, 2H, $SCH_2$), 4.5-5.0 (m, 2H, $CH_2N^{\oplus}$), 5.45 und 5.85 (jew. d, J=5Hz, 2 Lactam-H), 6.73 (t, J=72Hz, $CHF_2$), 7.5 (s, 1H, Thiazol). |
| 24 | Et | $-\overset{\oplus}{N}(Et)_2-Me$ | 1.33-1.63 (m, 9H, OEt und $NEt_2$), 2.90-3.95 (m, 8H, $SCH_2$, $NMeEt_2$), 4.10-5.10 (m, 4H, $CH_2N^{\oplus}$ und OEt), 5.50 und 5.91 (jew. d, J=5Hz, 2 Lactam-H), 7.4 (s, 1H, Thiazol). |
| 25 | Et | 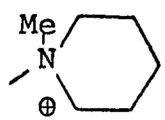 | 1.46 (t, J=7Hz, OEt), 1.60-2.40 (m, 6H, Piperidin-H), 3.30 (s, 3H, NMe), 3.30-3.95 (m, 6H, $SCH_2$ und 4 Piperidin-H), 4.15-5.0 (m, 4H, $CH_2N^{\oplus}$ und OEt), 5.50 und 5.93 (jew. d, J=5Hz, 2 Lactam-H), 7.43 (s, 1H, Thiazol). |

Tabelle 2 (Forts.)

| Beispiel | $R^2$ | A | $^1H$-NMR ($CF_3CO_2D$): δ (ppm) = |
|---|---|---|---|
| 26 | Me | 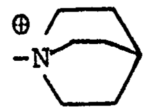 | 1.85-2.50 (m, 7H, Chinuclidin-H), 3.10-4.0 (m, 8H, $SCH_2$ und Chinuclidin-H), 4.26 (s, 3H, OMe), 4.56 (bs, 2H, $CH_2N^{\oplus}$), 5.43 und 5.86 (jew. d, J=5Hz, 2 Lactam-H), 7.43 (s, 1H, Thiazol). |
| 27 | Me | 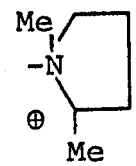 | 1.63 (d, J=7Hz, CH-$C\underline{H}_3$), 2.0-2.90 (m, 4H, Pyrrolidin-H), 3.0-4.10 (m, 8H, $SCH_2$, NMe und 3 Pyrrolidin-H), 4.26 (s, 3H, OMe), 4.10-4.83 ($AB_q$, J=17Hz, 2H, $CH_2N^{\oplus}$), 5.46 und 5.9 (jew. d, J=5Hz, 2 Lactam-H), 7.41 (s, 1H, Thiazol). |
| 28 | Me | 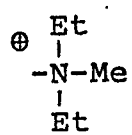 | 1.50 (t, J=7Hz, 6H, $NEt_2$), 3.20 (s, 3H, NMe), 3.30-4.0 (m, 6H, $SCH_2$ und $NEt_2$), 4.26 (s, 3H, OMe), 4.43-5.0 ($AB_q$, J=14Hz, 2H, $CH_2N^{\oplus}$), 5.46 und 5.90 (jew. d, J=5Hz, 2 Lactam-H), 7.43 (s, 1H, Thiazol). |
| 29 | $CH_2CH_2CH_3$ | $-\overset{\oplus}{N}Et_3$ | 1.01 (t, J=7Hz, 3H, OProp), 1.20-2.10 (m, 11H, $NEt_3$ und OProp), 3.10-4.0 (m, 8H, $SCH_2$ und $NEt_3$), 4.15-4.80 (m, 4H, $CH_2N^{\oplus}$ und $OCH_2$), 5.50 und 5.96 (jew. d, J=5Hz, 2 Lactam-H), 7.43 (s, 1H, Thiazol). |

Tabelle 2 (Forts.)

| Beispiel | $R^2$ | A | $^1H$-NMR in $CF_3CO_2D$: δ (ppm) = |
|---|---|---|---|
| 30 | Me | 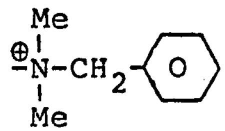 | 3.10-3.40 (m, 6H, $NMe_2$), 3.80 (bs, 2H, $SCH_2$), 4.23 (s, 3H, OMe), 4.35-5.0 (m, 4H, $CH_2N^{\oplus}$ und Benzyl-H), 5.50 und 5.90 (jew. d, J=5Hz, 2 Lactam-H), 7.20-7.70 (m, 6H, Thiazol-H und Aromaten-H). |
| 31 | Me | 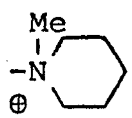 | 1.60-2.40 (m, 6H, Piperidin-H), 3.23 (s, 3H, NMe), 3.35-3.90 (m, 6H, $SCH_2$ und Piperidin-H), 4.23 (s, 3H, $OCH_3$), 4.46-5.10 ($AB_q$, J=14Hz, 2H, $CH_2N^{\oplus}$), 5.50 und 5.90 (jew. d, J=5Hz, 2 Lactam-H), 7.40 (s, 1H, Thiazol). |
| 32 | Et | 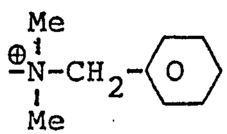 | 1.46 (t, J=7Hz, 3H, OEt), 3.10-3.40 (m, 6H, $NMe_2$), 3.80 (bs, 2H, $SCH_2$), 4.25-5.10 (m, 6H, $CH_2N^{\oplus}$, OEt und Benzyl-H), 5.50 und 5.93 (jew. d, J=5Hz, 2 Lactam-H), 7.35-7.65 (m, 6H, Thiazol-H und Aromaten-H). |
| 33 | Et | 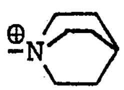 | 1.46 (t, J=7Hz, 3H, OEt), 1.80-2.50 (m, 7H, Chinuclidin-H), 3.30-4.0 (m, 8H, $SCH_2$ und Chinuclidin), 4.0-4.90 (m, 4H, $CH_2N^{\oplus}$ und OEt), 5.43 und 5.90 (jew. d, J=5Hz, 2 Lactam-H), 7.40(s, 1H, Thiazol). |

Analog Beispiel 6 werden die nachfolgend aufgeführten Beispiele als amorphe Feststoffe erhalten, die der allgemeinen Formel I mit $R^1$= Wasserstoff entsprechen und worin $R^2$, Z und A die in Tabelle 3 angegebene Bedeutung haben.

Tabelle 3

| Beispiel | $R^2$ | Z | A | $^1H$-NMR in $CF_2CO_2D$: δ (ppm) = |
|---|---|---|---|---|
| 34 | Me | CCl | $-\overset{\oplus}{N}Me_3$ | 3.36 (s, 9H, $NMe_3$), 3.83 (bs, 2H, $SCH_2$), 4.23 (s, 3H, OMe), 4.46-5.0 ($AB_q$, J=14Hz, 2H, $CH_2N^{\oplus}$), 5.46 und 5.96 (jew. d, J=5Hz, 2 Lactam-H). |
| 35 | Me | CBr | $-\overset{\oplus}{N}Me_3$ | 3.40 (s, 9H, $NMe_3$), 3.83 (bs, 2H, $SCH_2$), 4.30 (s, 3H, OMe), 4.50-5.06 ($AB_q$, J=14Hz, 2H, $CH_2N^{\oplus}$), 5.46 und 6.0 (jew. d, J=5Hz, 2 Lactam-H). |
| 36 | Me | CBr | $-\overset{\oplus}{N}Et_3$ | 1.50 (bt, J=6Hz, 9H, $NEt_3$), 3.10-3.95 (m, 8H, $SCH_2$ und $NEt_3$), 4.23 (s, 3H, OMe), 4.36-5.0 ($AB_q$, J=14Hz, 2H, $CH_2N^{\oplus}$), 5.46 und 5.96 (jew. d, J=5Hz, 2 Lactam-H). |
| 37 | Me | CCl | $-\overset{\oplus}{N}Et_3$ | 1.50 (bt, J=6Hz, 9H, $NEt_3$), 3.15-3.95 (m, 8H, $SCH_2$ und $NEt_3$), 4.23 (s, 3H, OMe), 4.40-5.0 ($AB_q$, J=7Hz, 2H, $CH_2N^{\oplus}$), 5.43 und 5.93 (jew. d, J=5Hz, 2 Lactam-H). |

0137440

## Beispiel 38

### 7-[2-(Aminothiazol-4-yl)-2-syn-(carboxymethyloxyimino)-acetamido]-3-trimethylammoniomethyl-ceph-3-em-4-carboxylat

Verfahren b

Lösung A

680 mg (2 mmol) 7-Amino-3-jodmethyl-ceph-3-em-4-carbonsäure werden in 30 ml N,N -Dimethylformamid suspendiert, 354 mg (6 mmol) Trimethylamin in 10 ml Dimethylformamid zugegeben und 2 Stunden bei Raumtemperatur gerührt.

Lösung B

963 mg (3 mmol) 2-(2-Aminothiazol-4-yl)-2-syn-(tert.-butyloxycarbonyl-methyl-oxyimino)-essigsäure, 618 mg (3 mmol) N,N'-Dicyclohexylcarbodiimid und 405 mg (3 mmol) 1-Hydroxybenzotriazolhydrat werden in 30 ml Dimethylformamid gelöst, 2 Stunden bei Raumtemperatur gerührt und der ausgefallene Niederschlag abfiltriert.

Lösung B wird bei 0° C zur Lösung A getropft und 16 Stunden bei Raumtemperatur gerührt. Die erhaltene Lösung wird in 250 ml Diäthyläther gegossen, der ausgefallene Niederschlag abgesaugt und mit Diäthyläther gewaschen. Der erhaltene braune Feststoff wird in 10 ml Trifluoressigsäure gelöst, 1 Stunde bei Raumtemperatur gerührt und nach Zugabe von 20 ml Toluol zur Trockene eingedampft. Der Rückstand wird in wenig Aceton/Wasser (3:1) gelöst und an Kieselgel mit Aceton/Wasser (3:1) chromatographiert. Gefriertrocknen der Produktfraktionen liefert 120 mg eines farblosen amorphen Feststoffes.

$^1$H-NMR in $CF_3CO_2D$: $\delta$ = 3.33 (s, 9H, $NMe_3$), 3.80 (bs, 2H, $SCH_2$), 4.50-4.86 ($AB_q$, J=14Hz, 2H, $CH_2N^{\oplus}$), 5.10 (s, 2H, $CH_2CO_2$), 5.46 und 5.96 (jew. d, J=5Hz, 2 Lactam-H), 7.43 ppm (s, 1H, Thiazol).

In Analogie zu Beispiel 38 werden die nachfolgend aufgeführten Verbindungen erhalten, die der allgemeinen Formel I mit $R^1$ = Wasserstoff und Z = CH entsprechen und als Reste $R^2$ und A die in Tabelle 4 angegebenen Substituenten tragen.

Tabelle 4

| Beispiel | $R^2$ | A | $^1H$-NMR in $CF_3CO_2D$: δ (ppm) = |
|---|---|---|---|
| 39 | $CH_2COOH$ | 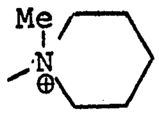 | 1.80-2.40 (m, 6H, Piperidin-H), 3.23 (s, 3H, NMe), 3.40-3.80 (m, 6H, $SCH_2$ und 4 Piperidin-H), 4.46-5.0 ($AB_q$, J=14Hz, 2H, $CH_2N^{\oplus}$), 5.50 und 5.90 (jew. d, J=5Hz, 2 Lactam-H), 7.4 (s, 1H, Thiazol), 5.06 (s, 2H, $CH_2COO$). |
| 40 | $CH_2COOH$ | 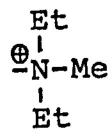 | 1.20-1.90 (m, 6H, $NEt_2$), 3.0-4.0 (m, 9H, $SCH_2$, $NEt_3$ und $NEt_2$), 4.43-5.0 ($AB_q$, J=14Hz, 2H, $CH_2N^{\oplus}$), 5.10 (s, 2H, $CH_2COO$), 5.50 und 5.93 (jew. d, J=5Hz, 2 Lactam-H), 7.43 (s, 1H, Thiazol). |
| 41 | 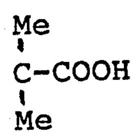 | 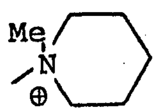 | 1.50-2.35 (m, 12H, $-OC(CH_3)_2$ und 6 Piperidin-H), 3.10-3.95 (m, 9H, NMe, $SCH_2$ und 4 Piperidin-H), 4.43-5.05 ($AB_q$, J=7Hz, 2H, $CH_2N^{\oplus}$), 5.46 und 5.96 (jew. d, J=5Hz, 2 Lactam-H), 7.40 (s, 1H, Thiazol. |
| 42 | $CH_2COOH$ | $^{\oplus}NEt_3$ | 1.50 (bt, J=7Hz, 9H, $NEt_3$), 3.10-3.95 (m, 8H, $SCH_2$ und $NEt_3$), 4.40-5.06 ($AB_q$, J=14Hz, 2H, $CH_2N^{\oplus}$), 5.10 (s, 2H, $OCH_2$), 5.5 und 5.96 (jew. d, J=5Hz, 2 Lactam-H). 7.46 (s, 1H, Thiazol). |

Analog Beispiel 1, Verfahren a) oder Beispiel 6 Verfahren b) werden die nachfolgend aufgeführten Verbindungen erhalten, die der allgemeinen Formel I mit $R^1$ = Wasserstoff und Z = CH entsprechen und als Reste $R^2$ und A die in Tabelle 5 angegebenen Substituenten tragen.

Tabelle 5

| Beispiel | $R^2$ | A | $^1H$-NMR in $CF_3CO_2D$: $\delta$(ppm)= |
|---|---|---|---|
| 43 | $CH_2CONH_2$ | $-\overset{\oplus}{N}Me_3$ | 3.36 (s, 9h, 3xMe); 3.81 (bs, 2H, $SCH_2$); 4.5-5.1 (ABq, J=14Hz, $CH_2N^{\oplus}$); 5.09 (bs, 2H, $OCH_2$); 5.49 und 5.94 (je ein d, J=5Hz, 2 Lactam-H); 7.44 (s, 1H, Thiazol). |
| 44 | $CH_2CONH_2$ | 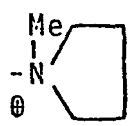 | 2.1-2.9 (m, 4H, Pyrrolidin-H); 3.23 (s, 3H, Me) 3.4 - 4.1 (m, 6H, $SCH_2$ und Pyrrolidin-H) 4.5-5.0 (m, 2H, $CH_2N^{\oplus}$); 5.11 (bs, 2H, $OCH_2$) 5.48 u. 5.97 (je ein d, J = 5Hz, 2Lactam-H); 7.47 (s, 1H, Thiazol |
| 45 | $CH_2CONH_2$ | 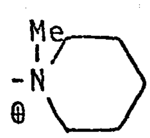 | 1.7-2.5 (m, 6H, Piperidin-H); 3.23 (s, 3H, Me); 3.4-4.0 (m, 6H, $SCH_2$ und Piperidin-H); 4.4-5.0 (m, 2H, $CH_2N^{\oplus}$); 5.0 (bs, 2H, $OCH_2$); 5.49 u. 5.95 (je ein d, J = 5Hz, 2Lactam-H); 7.46 (s, 1H, Thiazol) |

| Beispiel | $R^2$ | A | $^1H$-NMR in $CF_3CO_2D$: $\delta$ (ppm) = |
|---|---|---|---|
| 46 | $CH_2CONH_2$ | 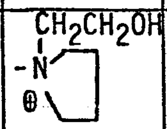 | 2.1-2.8 (m, 4H, Pyrrolidin-H) 2.7-4.6 (m, 10H, $SCH_2$, $CH_2CH_2OH$, 4Pyrrolidin-H); 4.6-5.1 (m, 2H, $CH_2N^{\oplus}$); 5.05 (bs, 2H, $OCH_2$); 5.46 und 5.93 (je ein d, J = 5Hz, 2Lactam-H); 7.44 (s, 1H, Thiazol). |
| 47 | $CH_2CONH_2$ | $\overset{\oplus}{N}(Me)_2C_2H_5$ | 1.57 (t, J=6Hz, 3H, NEt); 3.27 (bs, 6H, 2xMe); 3.4-3.9 (4H, q von NEt und bs von $SCH_2$ bei 3.82) 4.4-5.1 (AB, J=14Hz, 2H, $CH_2N^{\oplus}$);5.10 (bs, 2H, $OCH_2$) 5.48 und 5.94 (je ein d, J=5Hz, 2Lactam-H); 7.44 (s, 1H,Thiazol) |
| 48 | $-CH_2-C{\equiv}CH$ | $-\overset{\oplus}{N}ME_3$ | 2.6 (t; J=1.8 Hz, 1H, Propargyl-CH); 3.36 (bs, 9H, $NMe_3$); 3.81 (bs, 2H, $SCH_2$); 4.55-5.2 (4H, AB von $CH_2\overset{\oplus}{N}$ mit J=13Hz und d von Propargyl-$CH_2$ bei 4.98); 5.49 und 5.9 (jew. d, J=5Hz, 2Lactam-H); 7.43 (s, 1H, Thiazol). |
| 49 | $-CH_2-C{\equiv}CH$ | $\overset{\oplus}{N}-Et_3$ | 1.28-1.75 (m, 9H, $NEt_3$); 2.6 (t, J=18Hz, 1H, Propargyl-CH), 3.05-4.0 (m, 8H, $SCH_2$ und $NEt_3$); 4.4-5.15 (4H, AB von $CH_2\overset{\oplus}{N}$ mit J=14Hz und d von Propargyl-$CH_2$ bei 4.99); 5.48 und 5.9 (jew. d, J=5Hz, 2Lactam-tam-H); 7.44 (s, 1H, Thiazol). |

| Beispiel | $R^2$ | A | $^1H$-NMR in $CF_3CO_2D$: $\delta$ (ppm) = |
|---|---|---|---|
| 50 | $-CH_2-C{\equiv}CH$ | 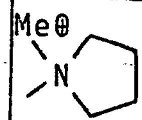 | 2.1-2.85 (m, 5H, 4Pyrrolidin-H und Propargyl-CH); 3.23 (S, 3H, NMe); 3.4-4.15 (m, 6H, $SCH_2$ und 4 Pyrrolidin-H); 4.5-5.05 (m, 4H, $CH_2\overset{\oplus}{N}$ und Propargyl-$CH_2$); 5.46 und 5.9 (jew. d, J=5Hz, 2 Lactam-H); 7.43 (s, 1H, Thiazol). |
| 51 | $-CH_2-CH{=}CH_2$ | $-\overset{\oplus}{N}Me_3$ | 3.38 (bs, 9H, $NMe_3$); 3.8 (bs, 2H, $SCH_2$); 4.4-6.05 (m, 9H, $CH_2\overset{\oplus}{N}$, 5 Allyl-H und 2 Lactam-H bei 5.45 und 5.92); 7.44 (s, 1H, Thiazol) |
| 52 | $-CH_2-CH{=}CH_2$ | $-\overset{\oplus}{N}Et_3$ | 1.54 (bt, J = 6Hz, 9H, $NEt_3$); 3.15-3.95 (m, 8H, $SCH_2$ und $NEt_3$); 4.45-6.1 (m, 9H, $CH_2\overset{\oplus}{N}$, 5 Allyl-H und 2 Lactam-H bei 5.48 und 5.92), 7.44 (s, 1H, Thiazol). |
| 53 | $-CH_2-CH{=}CH_2$ | 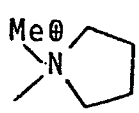 | 2.1-2.85 (m, 4 Pyrrolidin-H); 3.26 (bs, 3H, NMe); 3.4-4.1 (m, 6H, $SCH_2$ und 4Pyrrolidin-H); 4.5-6.08 (m, 9H, $CH_2\overset{\oplus}{N}$, 5 Allyl-H und 2 Lactam-H bei 5.46 und 5.92), 7.44 (s, 1H, Thiazol). |

PATENTANSPRÜCHE:

1. Cephalosporinderivate der allgemeinen Formel I

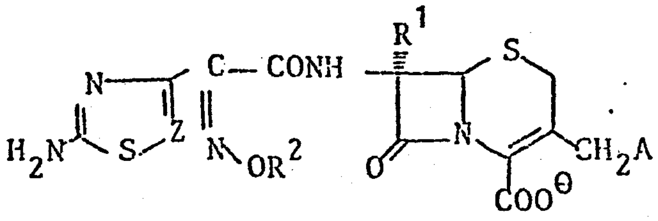

(I)

und deren physiologisch verträgliche Säureadditionssalze, worin

$R^1$ Wasserstoff oder Methoxy,

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-(\overset{R^3}{\underset{R^4}{C}})_m-R^5$, worin m und n jeweils für 0 oder 1 steht

und

$R^3$ und $R^4$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfachsubstituiert sein können,

$R^5$ eine COOH-; CN- oder $CONH_2$-Gruppe,

A eine gegebenenfalls substituierte aliphatische oder cyclische Ammoniogruppe bedeutet und

Z für CH, CF, CCl CBr oder N steht, und in der die $R^2O$-Gruppe in syn-Position steht.

2. Verfahren zur Herstellung von Cephemverbindungen der Formel I und deren physiologisch verträglichen Säureadditionssalzen, dadurch gekennzeichnet, daß man

a) eine Verbindung der allgemeinen Formel II

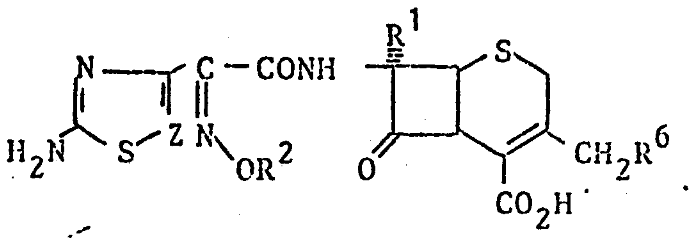

(II)

oder deren Salze, worin $R^1$, $R^2$ und Z die in Formel I genannte Bedeutung haben und $R^6$ eine durch diejenige Base, die den Resten A der Formel I entsprechen, austauschbare Gruppe bedeutet, mit dieser Base umsetzt und

α) eine gegebenenfalls vorhandene Schutzgruppe abspaltet und

ß) falls erwünscht, , das erhaltene Produkt in ein physiologisch verträgliches Säureadditionssalz überführt.

oder

b) eine Verbindung der allgemeinen Formel III

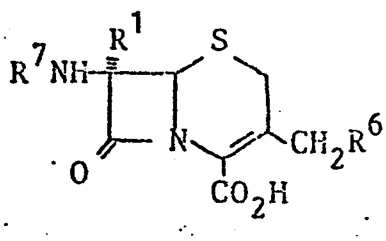

(III)

worin $R^1$ und $R^6$ die vorstehend für die Formel II genannte Bedeutung haben und $R^7$ für Wasserstoff oder eine Aminoschutzgruppe steht, mit der Base, die dem in Formel I definierten Rest A zugrunde liegt, umsetzt unter Bildung der Verbindung der allgemeinen Formel IV,

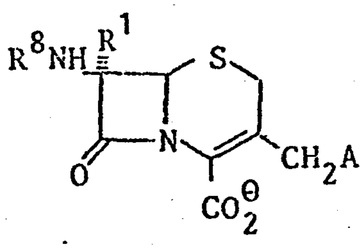

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

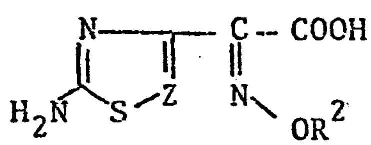

(V)

worin $R^2$ und Z die genannte Bedeutung besitzen, oder mit einem an der Carbonylgruppe aktivierten Derivat dieser Verbindung umsetzt und

α) eine gegebenenfalls vorhandene Schutzgruppe abspaltet und

β) falls erwünscht, das erhaltene Produkt der allgemeinen Formel I in ein physiologisch verträgliches Säureadditionssalz überführt.

3. Verfahren gemäß Anspruch 2, dadurch gekennzeichnet, daß der nucleophile Austausch des Substituenten $R^6$ in Gegenwart der dem Rest A zugrundeliegenden Base und von Tri-$C_1$-$C_4$-alkyljodsilan erfolgt.

4. Verfahren nach Anspruch 3, dadurch gekennzeichnet, daß das Tri-$C_1$-$C_4$-alkyljodsilan Trimethyl- oder Triethyljodsilan ist.

5. Gegen bakterielle Infektionen wirksame pharmazeutische Präparate, gekennzeichnet durch einen Gehalt an Cephemderivaten der allgemeinen Formel I.

6. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

7. Verwendung von Cephemderivaten der allgemeinen Formel I zur Bekämpfung bakterieller Infektionen.

8. Cephemverbindungen gemäß Anspruch 1, dadurch gekennzeichnet, daß für den Fall, daß Z für CH und $R^1$ für Wasserstoff steht,

α) A nicht bedeuten kann Tri-$C_1$-$C_4$-Alkylammonium und $R^2$ nicht sein kann

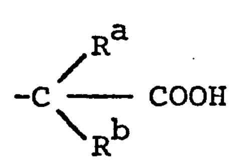

mit $R^a$ und $R^b$ = $C_1$-$C_4$-Alkyl, $C_3$-$C_7$-Cycloalkyliden oder

ß) A nicht bedeuten kann 1-Methyl-1-pyrrolidinium und $R^2$ nicht sein kann $C_1$-$C_4$-Alkyl, Allyl, 2-Butenyl, 3-Butenyl oder

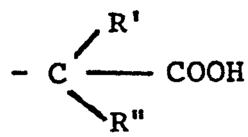

mit R' und R" = Wasserstoff, Methyl, Äthyl oder auch zusammen $C_3$-$C_5$-Cycloalkyliden.

Patentansprüche für Österreich:

1. Verfahren zur Herstellung von Chephalosporinderivaten der allgemeinen Formel I

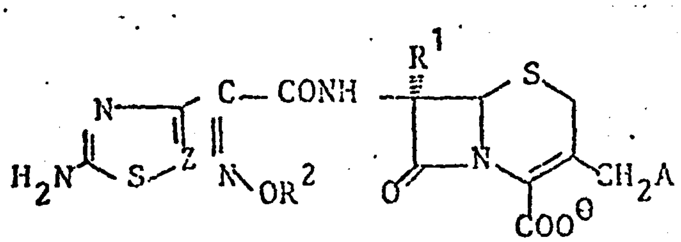

(I)

und deren physiologisch verträglichen Säureadditionssalzen, worin

$R^1$ Wasserstoff oder Methoxy,

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-(\overset{R^3}{\underset{R^4}{C}})_m-R^5$, worin m und n jeweils für 0 oder 1 steht

und

$R^3$ und $R^4$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfachsubstituiert sein können,

$R^5$ eine COOH-; CN- oder $CONH_2$-Gruppe,

A eine gegebenenfalls substituierte aliphatische oder cycl ische Ammoniogruppe bedeutet und

Z für CH, CF, CCl CBr oder N steht, und in der die $R^2O$-Gruppe in syn-Position steht,

dadurch gekennzeichnet, daß man

a) eine Verbindung der allgemeinen Formel II

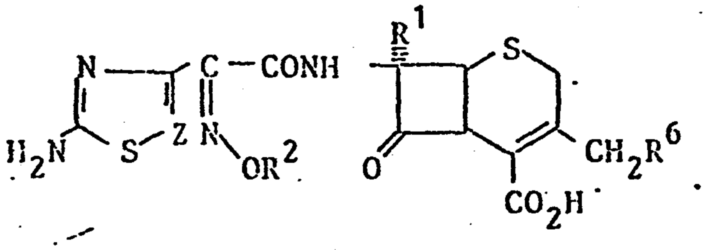

(II)

oder deren Salze, worin $R^1$, $R^2$ und Z die in Formel I genannte Bedeutung haben und $R^6$ eine durch diejenige Base, die den Resten A der Formel I entsprechen, austauschbare Gruppe bedeutet, mit dieser Base umsetzt und

α) eine gegebenenfalls vorhandene Schutzgruppe abspaltet und

ß) falls erwünscht, , das erhaltene Produkt in ein physiologisch verträgliches Säureadditionssalz überführt.

oder

b) eine Verbindung der allgemeinen Formel III

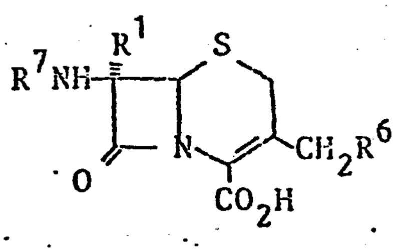

(III)

worin $R^1$ und $R^6$ die vorstehend für die Formel II genannte Bedeutung haben und $R^7$ für Wasserstoff oder eine Aminoschutzgruppe steht, mit der Base, die dem in Formel I definierten Rest A zugrunde liegt, umsetzt unter Bildung der Verbindung der allgemeinen Formel IV,

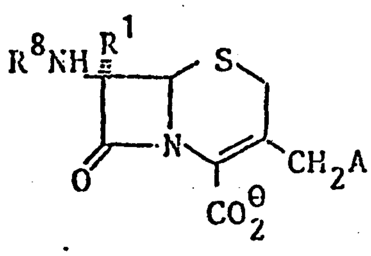

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

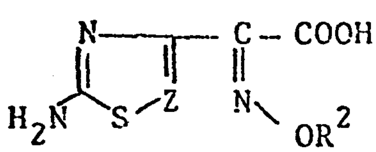

(V)

worin $R^2$ und Z die genannte Bedeutung besitzen, oder mit einem an der Carbonylgruppe aktivierten Derivat dieser Verbindung umsetzt und

γ) eine gegebenenfalls vorhandene Schutzgruppe abspaltet und

ß) falls erwünscht, das erhaltene Produkt der allgemeinen Formel I in ein physiologisch verträgliches Säureadditionssalz überführt.

2. Verfahren gemäß Anspruch 1, dadurch gekennzeichnet, daß der nucleophile Austausch des Substituenten $R^6$ in Gegenwart der dem Rest A zugrundeliegenden Base und von Tri-$C_1$-$C_4$-alkyljodsilan erfolgt.

3. Verfahren nach Anspruch 2, dadurch gekennzeichnet, daß das Tri-$C_1$-$C_4$-alkyljodsilan Trimethyl- oder Triethyljodsilan ist.

4. Verfahren gemäß Anspruch 1, wobei in den Cephemverbindungen der Formel I für den Fall, daß Z für CH und $R^1$ für Wasserstoff steht,

α) A nicht bedeuten kann Tri-$C_1$-$C_4$-Alkylammonium und $R^2$ nicht sein kann

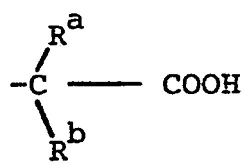

mit $R^a$ und $R^b$ = $C_1$-$C_4$-Alkyl, $C_3$-$C_7$-Cycloalkyliden oder

ß) A nicht bedeuten kann 1-Methyl-1-pyrrolidinium und $R^2$ nicht sein kann $C_1$-$C_4$-Alkyl, Allyl, 2-Butenyl, 3-Butenyl oder

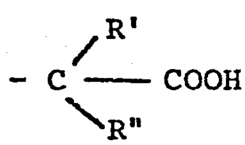

mit R' und R" = Wasserstoff, Methyl, Äthyl oder auch zusammen $C_3$-$C_5$-Cycloalkyliden.